# FASCICULE DE BREVET EUROPEEN

(11) **EP 3 534 980 B1**
(45) Date de publication et mention de la délivrance du brevet: **02.09.2020**
(21) Numéro de dépôt: 17803824.6
(22) Date de dépôt: 07.11.2017
(51) Int. Cl.: A61L 27/36, A61L 27/38

(54) **GUIDE IMPLANTABLE POUR LA REGENERATION TISSULAIRE**
IMPLANTIERBARE FÜHRUNG ZUR GEWEBEREGENERATION
IMPLANTABLE GUIDE FOR TISSUE REGENERATION

(30) Priorité: 07.11.2016 FR 1660758
(43) Date de publication de la demande: 11.09.2019
(73) Titulaire: TBF Genie Tissulaire (TBF), 69780 Mions (FR)
(72) Inventeur: BARNOUIN, Laurence, 38460 Vénérieu (FR)
(74) Mandataire: Tripoz, Inès
(86) Numéro de dépôt international: PCT/EP2017/078513
(87) Numéro de publication internationale: WO 2018/083353

(56) Documents cités:
- US-B1- 7 294 144
- CROUZIER T ET AL: "Inverted human umbilical arteries with tunable wall thickness for nerve regeneration", JOURNAL OF BIOMEDICAL MATERIALS RESEARCH, WILEY, NEW YORK, NY, US, vol. 89, 9 juillet 2008 (2008-07-09), pages 818-828, XP002629943, ISSN: 0021-9304, DOI: 10.1002/JBM.A.32103 [extrait le 2008-07-09] cité dans la demande

## Description

La présente invention concerne les guides implantables, plus particulièrement, aux dispositifs médicaux d'origine biologique. La présente invention est notamment destinée à la régénération tissulaire de tissus sectionnés tels que des nerfs, des ligaments ou des tendons par pontage entre les deux extrémités tissulaires sectionnées.

Lors d'un traumatisme de certains tissus biologiques, une déchirure ou section peut se produire, provoquant ainsi un éloignement spatial entre les deux extrémités du tissu lésé. Ceci arrive notamment lorsqu'un nerf du système nerveux périphérique, un tendon, ou un ligament est sectionné ou déchiré.

Ce type de blessures est en général traité par des techniques de sutures consistant à rapprocher les deux extrémités (dites extrémité proximale et extrémité distale) du tissu lésé avant de les suturer bout à bout. Toutefois, ces techniques ne sont utilisables que lorsque la distance entre les extrémités distale et proximale n'est pas trop importante, afin de permettre la suture directe proximale-distale. Lorsque cette distance est trop importante pour permettre cette suture directe, une procédure d'autogreffe peut être utilisée. Cette technique présente comme inconvénient la nécessité de procéder à une opération chirurgicale invasive chez le patient, afin de prélever un greffon aux dimensions appropriées visant à réaliser le pontage entre les extrémités proximale et distale du tissu lésé.

Un autre inconvénient est que la suture directe ou la suture par pontage d'autogreffe présente parfois des complications ne permettant pas la régénération des tissus, ou toutefois, une régénération indésirable. Il peut, par exemple, advenir que du tissu cicatriciel issu du site de l'intervention chirurgicale interfère avec une régénération tissulaire satisfaisante entre les extrémités proximale et distale. Ceci est d'autant plus problématique dans le cas de la régénération d'un nerf. En effet, il arrive que la repousse des axones soit interrompue par ce phénomène, la conséquence étant que les fonctions sensorielles et/ou motrices ne sont pas restaurées.

Ainsi, des stratégies destinées à protéger le site biologique de la lésion de toute forme d'infiltration par des tissus fibreux ont été développées. Par exemple, par utilisation de bandages ou de tubes biocompatibles pour envelopper les sutures. Dans le domaine de la régénération des nerfs, des solutions de guides implantables destinés tant à protéger qu'à guider la repousse du nerf sectionné ont été développées.

Dans « Stratégies for regeneration of components of nervous system: scaffolds, cells and biomolecules », Tian *et al.* retracent l'évolution de ces biomatériaux d'origine synthétique. Le silicone fut à l'origine le matériau employé pour le développement de ces guides de régénération, celui-ci étant inerte, facilement disponible et, dans une certaine mesure, élastique. Mais, en conditions cliniques, de nombreux patients se plaignirent d'irritations au site d'intervention, nécessitant une nouvelle opération pour ôter le dispositif. Le polytetrafluoroéthylène (PTFE) fut introduit comme polymère permettant de ponter des intervalles jusqu'à 40 mm entre les extrémités proximale et distale. Malheureusement, il a été remarqué que le PTFE compressait le nerf, conduisant à une altération de la régénération de celui-ci, tout en causant des irritations chez le patient. Enfin, le collagène, protéine structurelle abondante dans de nombreux tissus chez l'animal, notamment prévalant dans les matrices extracellulaires, devint un polymère de choix pour réaliser des guides de régénération des nerfs. Ce polymère ne présentant pas les défauts de ses prédécesseurs.

On peut citer par exemple le brevet US 5,019,087 divulguant un dispositif tubulaire implantable dont la paroi est constituée d'une matrice de collagène de Type I et de laminine. La lumière de ce guide est exempte d'une quelconque matrice au sein de la lumière du canal.

Le brevet US 6,716,225 divulgue un dispositif tubulaire implantable en matériaux biocompatibles et bio-résorbables tels que les collagènes, les polysaccharides, ou d'autres protéines comme la fibrine ou l'élastine. La lumière du canal peut inclure des microtubes, des filaments, ou une matrice poreuse définissant un microsystème de guidage permettant d'améliorer l'adhérence cellulaire et d'orienter la migration cellulaire au sein du guide.

Des dispositifs à base de polymères synthétiques ont également été développés. On peut citer en exemple le brevet EP 0 327 022 qui divulgue un dispositif tubulaire implantable, constitué par exemple d'acide polyglycolique ou d'un copolymère de glycolide et de carbonate de triméthylène.

Tous ces dispositifs constitués de matériaux synthétiques ont comme inconvénient majeur de pouvoir présenter des problèmes de compatibilité biologique.

Des guides pour la régénération de nerfs créés à partir de structures biologiques tissulaires existent également. On peut citer le brevet US 6,241,981 qui divulgue un dispositif tubulaire implantable obtenu à partir du tissu d'une sous-muqueuse originaire d'un vertébré à sang chaud. De manière préférée, la sous-muqueuse est la sous-muqueuse de l'intestin. Celle-ci est une matrice naturelle de collagène comprenant des molécules d'intérêt comme des glycoprotéines, des protéoglycanes, ou des glycosaminoglycanes dans leurs configurations et concentrations naturelles. Le dispositif peut être combiné avec des facteurs de croissance de types VEGF, NGF ou FGF. Une fois la sous-muqueuse mise en forme sous la forme d'un tube, la structure peut également être remplie d'un extrait de tissu de sous-muqueuse fluidisé. Ce guide pour la régénération de nerfs peut être déshydraté par lyophilisation, et réhydraté avant implantation.

Dans « Inverted human umbilical arteries with tunable wall thicknesses for nerve regeneration », Journal of Biomedical Materials Research Part A, p.818-828, Crouzier *et al.* divulguent un guide nerveux tubulaire dérivé d'une artère du cordon ombilical dont la paroi est retournée. Ce retournement est décrit comme présentant les avantages d'éviter que le guide s'effondre sur lui-même, ainsi que de permettre l'exposition du neurone en croissance au sein du guide à une substance riche en collagène qui est la gelée de Wharton.

Un autre dispositif implantable, obtenu à partir d'un vaisseau issu du cordon ombilical de mammifère est également décrit dans le brevet US 7,294,144. Ledit vaisseau est séparé de la gelée de Wharton et est lyophilisé. La destination du canal obtenu selon ce procédé est la greffe vasculaire. La greffe nécessite une réhydratation du tissu avant implantation.

Les guides pour la régénération tissulaire, issus des tissus biologiques, présentent un risque de transmission d'une infection d'un donneur vers un receveur, ou d'un risque de contamination lors de la collecte du tissu à l'accouchement, lors des traitements préparatoires du tissu, ou lors du transport de ce tissu. De fait, un procédé efficace tant de désinfection que de conservation de ces tissus est vital pour la sécurité des patients.

Tous ces dispositifs présentent certains inconvénients et notamment des problèmes de compatibilité biologique, de conditionnement, de conservation et de mise à disposition des praticiens, de rigidité à la mise en place lors de l'intervention, et potentiellement de viro-inactivation. Soit que le traitement n'est pas suffisamment efficace, soit qu'il est trop long et/ou destructeur vis-à-vis du matériau naturel.

Le défaut de rigidité des dispositifs implantables, obtenus à partir de vaisseaux sanguins, est connu pour avoir pour conséquence un risque de collapsus du guide pour la régénération tissulaire (Konofaos et al. « Nerve repair by means of tubulization: past, present, future. » J reconstr Microsurg 2013; 29(03): 149-164; et Mohammadi et al. « Comparison of repair of peripheral nerve transection in predegenerated muscle with and without a vein graft » BMC Neurol. 2016; 16: 237).

Le maintien de la forme du guide implantable non collabable pour la régénération tissulaire est donc important pour prévenir ce risque de collapsus. En effet, un collapsus du guide implique une diminution de la capacité du tissu d'intérêt à coloniser la lumière dudit guide implantable non collabable lors de son processus biologique de régénération.

Un objectif de la présente invention est donc de proposer un guide implantable non collabable pour la régénération tissulaire, et son procédé de préparation, permettant de résoudre les problèmes présentés par les dispositifs ci-dessus décrits.

La présente invention consiste en un guide implantable non collabable pour la régénération tissulaire, constitué d'un segment d'artère ou de veine ombilicale dont la paroi est retournée, caractérisé en ce qu'il est viro-inactivé et lyophilisé, implantable sans réhydratation préalable, et permet de résoudre l'intégralité de ces problèmes.

La présente invention concerne également un procédé de préparation dudit guide implantable non collabable pour la régénération tissulaire.

Elle concerne également un procédé d'implantation qui comprend la mise en place du guide implantable non collabable pour la régénération tissulaire sans étape préalable d'hydratation.

Définitions utilisées dans la présente demande.

Certaines expressions ou mots peuvent être utilisés dans la présente demande pour indiquer des éléments techniques, faits, propriétés ou caractéristiques. Leur signification est donnée ci-après :
- par « vaisseau issu du cordon ombilical », on entend un vaisseau sanguin, qui peut être la veine ou l'une des deux artères, les trois vaisseaux présents dans le cordon ombilical de mammifère étant exploitables selon l'invention.
- par « vaisseau retourné » ou « paroi retournée », on entend que le tissu initial, qui est un vaisseau sanguin, est retourné sur lui-même de telle sorte que la paroi constitutive de la lumière du vaisseau avant retournement devient la paroi externe du vaisseau, et que la paroi qui était la paroi externe du vaisseau avant retournement devient la nouvelle paroi de la nouvelle lumière du vaisseau.
- par « segment », on entend une fraction du tissu d'origine, qui est un vaisseau sanguin, et qui a été sectionné en deux extrémités de telle sorte que la fraction présente la forme d'un cylindre. Ce cylindre est ainsi caractérisé par une paroi qui est la paroi du vaisseau sanguin, et de deux extrémités circulaires ouvertes permettant l'accès à la lumière dudit cylindre.
- par « régénération », on entend la capacité des tissus vivants à se reconstituer après destruction. Par exemple après une blessure de type sectionnement, écrasement ou déchirement. Dans la présente invention, les tissus considérés sont les nerfs du système nerveux périphérique, les ligaments et les tendons.
- par « viro-inactivation », on entend une technique qui permet de réduire fortement ou totalement, et définitivement, la capacité d'action des virus. Ceux-ci, définis comme inactivés, perdent leurs capacités pathogéniques et de réplication par une diminution de leur population de 4 log lors des titrages résiduels qui suivent une ou deux étapes chimiques indépendantes, que ce soit sur des virus enveloppés, ou non enveloppés, ADN ou ARN.
- par « lyophilisation », on entend une technique visant à dessécher un produit préalablement surgelé par sublimation. Plus précisément, le liquide à ôter du produit est dans un premier temps transformé en glace par congélation ; puis par une dessiccation primaire, sous vide, la glace est sublimée ; enfin par une dessiccation secondaire, les molécules d'eau à la surface du produit sont extraites par désorption.
- par « ne pas endommager l'intégrité structurelle, fonctionnelle et biologique du tissu », on entend que le guide pour la régénération tissulaire obtenu selon le procédé présente une conservation suffisante des molécules biologiques assurant la structure mécanique du tissu, tels que le collagène de divers types, les protéoglycanes, et l'élastine, ainsi que des molécules biologiquement actives, comme par exemple les hormones, enzymes, facteurs de croissance, afin d'assurer une utilisation ultérieure du produit. Ainsi, selon ces paramètres, le produit obtenu selon le procédé présente des propriétés biologiques similaires ou quasi-similaires au tissu initial avant traitement. Dans la présente invention, ces molécules sont par exemple le collagène IV et l'élastine ; ainsi que les facteurs de croissance de type NGF (Nerve Growth Factor), FGF (Fibroblast Growth Factor), TGF (Transforming Growth Factor).
- par « conditionnement », on entend tout type d'action visant à placer le guide pour la régénération tissulaire dans une condition d'emballage permettant sa conservation à l'air libre et à température ambiante pendant au moins cinq ans.
- par « alcools », on entend tout composé organique dont l'un des carbones est lié à un groupe hydroxyle (-OH).
- par « peroxydes », on entend tout composé chimique contenant un groupe fonctionnel de formule générale R-O-O-R' (deux atomes d'oxygène voisins liés entre eux) où R et R' sont des atomes d'hydrogène ou des radicaux alkyls quelconques.
- par « non collabable », on entend que le guide pour la régénération tissulaire selon l'invention, est caractérisé par une déformabilité limitée en réponse à l'application d'une force mécanique et que notamment il ne présente pas de collapsus. En d'autres termes, l'aire de la section droite est sensiblement constante quelles que soient les pressions exercées. Plus précisément, le guide implantable pour la régénération tissulaire selon l'invention, qui a une forme tubulaire, ne s'affaisse pas lorsqu'il est pris par exemple par l'intermédiaire d'une pince, il conserve sa forme. En effet, le guide implantable pris en l'une de ses extrémités, par exemple par l'intermédiaire d'une pince, et tenu horizontalement, conserve son aspect tubulaire rectiligne et ne s'effondre pas verticalement de par la force de gravité exercée. A la différence, une veine ou artère utilisée en milieu humide par exemple après prélèvement lors d'une autogreffe ne peut être considérée comme non collabable : prise par exemple en l'une de ses extrémités ou en son centre, elle se déforme par l'effet de la gravité et sa forme tubulaire rectiligne n'est pas conservée.Le guide pour la régénération tissulaire selon l'invention présente néanmoins une souplesse suffisante afin de s'adapter aux gestes techniques chirurgicaux du praticien. Le guide selon l'invention peut notamment prendre une forme courbe lorsqu'il est glissé par exemple sur une aiguille courbe ou sur un nerf lors d'une technique chirurgicale de manchonnage.

Cette souplesse du guide implantable est suffisante pour notamment accompagner les mouvements d'un patient ayant par exemple reçu pour greffe un guide implantable selon l'invention en manchonnage d'un nerf à régénérer. Par exemple, le guide implantable selon l'invention utilisé dans la réparation d'un nerf collatéral palmaire de la main permettra au patient de mouvoir ses doigts sans résistance manifeste. En effet, le guide implantable selon l'invention est non collabable mais possède une souplesse suffisante pour accompagner les déformations mécaniques induites par le geste du praticien ou le mouvement du patient après implantation sans provoquer une tension mécanique au niveau du site d'implantation.

Il est en effet connu que des neurotubes, notamment synthétiques ou en collagène, présentent comme inconvénient que leur structure tubulaire est très peu déformable, ce qui entraîne notamment un risque d'extrusion du matériau par la cicatrice lors d'un mouvement du patient greffé.

Le guide pour la régénération tissulaire selon l'invention a donc pour caractéristique d'être « non collabable », en ce qu'il n'est ni mou ou trop souple comme peut l'être par exemple une veine ou une artère humide, ni pas ou peu déformable comme peut l'être par exemple un neurotube synthétique ou en collagène.
- par « enrobage » ou « enveloppement », on entend une technique chirurgicale visant à envelopper un tissu lésé par un matériau de greffe utilisé par enveloppement d'un tissu lésé dont la forme est longitudinale. Ainsi, une fois installé sur le site d'implantation, le matériau de greffe a une forme tubulaire entourant le tissu lésé longitudinal. Par exemple, un nerf lésé sera enrobé ou enveloppé par le guide pour la régénération tissulaire selon l'invention après ouverture longitudinale du guide. Le guide ouvert vient longitudinalement envelopper ou enrober le nerf lésé et le protège notamment pour éviter les adhérences avec les tissus environnants tout en lui fournissant un milieu adéquat pour assurer sa régénération.
- par « manchonnage », on entend une technique chirurgicale visant à recouvrir un tissu lésé par un matériau d'allogreffe de forme tubulaire appelé « manchon ». Par exemple, lorsqu'un nerf est sectionné, le manchon est « enfilé en chaussette » sur l'une des extrémités du nerf. Le nerf est alors suturé par des points épi-périneuraux, puis le manchon est glissé au-delà la suture nerveuse et positionné de part et d'autre de celle-ci avec un débord équidistant de la ligne de suture.

Le manchonnage peut également être défini comme une suture entre les deux extrémités du manchon, et le manchonnage ne couvre ainsi que les deux extrémités du tissu lésé.

Le guide implantable non collabable pour la régénération tissulaire selon l'invention est ci-après décrit.

Le guide implantable non collabable pour la régénération tissulaire, est constitué d'un segment d'artère ou de veine ombilicale dont la paroi est retournée, caractérisé en ce qu'il est viro-inactivé et lyophilisé.

Le guide implantable non collabable est caractérisé en ce qu'il est implantable sans réhydratation préalable. En effet, le guide implantable non collabable est notamment lyophilisé avec une lumière ouverte, caractéristique qui permet au produit d'être aisément conservable, mais lui confère également une rigidité structurelle. Cette rigidité du guide implantable non collabable permet notamment sa manipulation aisée par l'utilisateur lors d'une intervention chirurgicale.

Le guide implantable non collabable pour la régénération tissulaire est également caractérisé en ce que ladite rigidité du guide permet une certaine flexibilité pour pouvoir glisser le long du nerf sectionné et plier lors de son implantation, ainsi que lors des mouvements moteurs du patient ayant subi l'implantation, tout en étant suffisamment rigide pour diminuer les risques de collapsus in vivo.Le guide implantable non collabable est caractérisé en ce qu'il comprend dans sa lumière de la gelée de Wharton.

La demanderesse a mis en évidence, par l'observation de coupes histologiques, qu'un guide implantable non collabable pour la régénération tissulaire selon l'invention, constitué d'un segment d'artère ou de veine ombilicale dont la paroi est retournée, comprenant dans sa lumière de la gelée de Wharton, caractérisé en ce qu'il est viro-inactivé et lyophilisé, présente une structure poreuse adjointe à la surface interne du guide particulièrement appréciée en régénération tissulaire.

En effet, il a été remarqué par la demanderesse que la gelée de Wharton, comprise dans la lumière du guide implantable non collabable selon l'invention, était caractérisée par une structure histologique différente d'un produit qui n'est pas viro-inactivé et lyophilisé.

La gelée de Wharton, comprise dans la lumière du guide implantable non collabable est une structure fibreuse et longitudinale. Ces caractéristiques structurelles, après viro-inactivation et lyophilisation, induisent un réseau poreux longitudinal le long de la surface interne du guide implantable non collabable selon l'invention. Cette structure longitudinale poreuse est particulièrement appréciée en régénération tissulaire guidée en ce que le tissu qui se régénère rencontre une résistance réduite lors de son développement biologique, et en ce que sa régénération est orientée dans un axe favorable au sein de ce réseau poreux longitudinal.

Cette rigidité du guide implantable non collabable pour la régénération tissulaire selon l'invention permet donc la diminution des risques de collapsus, tout en fournissant au tissu destiné à se régénérer au sein dudit guide un réseau poreux longitudinal propice à son développement biologique.

Le guide implantable non collabable est caractérisé en ce que l'épaisseur de la gelée de Wharton est comprise entre 0,3 et 2 millimètres.

Le guide implantable non collabable est caractérisé en ce que l'épaisseur de la gelée de Wharton est comprise entre 0,3 et 1 millimètre lorsqu'il est constitué d'un segment d'artère ombilicale.

Le guide implantable non collabable est caractérisé en ce que l'épaisseur de la gelée de Wharton est comprise entre 1 et 2 millimètres, lorsqu'il est constitué d'un segment de veine ombilicale.

Le guide implantable non collabable est caractérisé en ce que le diamètre de la lumière du segment est compris entre 1 et 7 millimètres.

Le guide implantable non collabable est caractérisé en ce que le diamètre de la lumière du segment est compris entre 1 et 2 millimètres lorsqu'il est constitué d'un segment d'artère ombilicale.

Le guide implantable non collabable est caractérisé en ce que le diamètre de la lumière du segment est compris entre 2 et 7 millimètres lorsqu'il est constitué d'un segment de veine ombilicale.

Le guide implantable non collabable est caractérisé en ce que la longueur du segment est comprise entre 1 et 12 centimètres.

Le guide implantable non collabable est caractérisé en ce que la longueur du segment est comprise entre 1 et 6 centimètres lorsqu'il est constitué d'un segment d'artère ombilicale.

Le guide implantable non collabable est caractérisé en ce que la longueur du segment est comprise entre 2 et 12 centimètres lorsqu'il est constitué d'un segment de veine ombilicale.

Le guide implantable non collabable est caractérisé en ce qu'il est destiné à être utilisé comme matériau d'allogreffe choisie dans le groupe constitué par la greffe de guide de régénération nerveuse, la greffe de guide de régénération tendineuse, la greffe de guide de régénération ligamentaire.

Le guide implantable non collabable peut être utilisé en tant qu'allogreffe choisie dans le groupe constitué par la réparation d'un nerf du système nerveux périphérique, la réparation du tendon, la réparation du ligament.

La présente invention concerne également un procédé de préparation d'un guide implantable non collabable pour la régénération tissulaire, constitué d'un segment d'artère ou de veine ombilicale dont la paroi est retournée, caractérisé en ce qu'il est viro-inactivé et lyophilisé.

Le guide implantable non collabable obtenu selon le procédé est caractérisé en ce qu'il est implantable sans réhydratation préalable.

Le tissu biologique destiné à être traité selon le procédé décrit dans la présente invention, et destiné à former un guide pour la régénération tissulaire est un vaisseau issu d'un cordon ombilical. Ce cordon ombilical est obtenu après l'accouchement d'un donneur proprement informé et consentant en accord avec les exigences des directives Européennes et la Loi Bioéthique. Par exigence sanitaire relative aux dons de tissus et de cellules d'origine humaine, une qualification socio-clinique du donneur et biologique en amont du donneur est systématique. Cette qualification implique une recherche de virus HIV, hépatites B, C, HTLV et de la bactérie tréponème pâle responsable de la syphilis. Le cordon ombilical est avantageusement placé dans une boîte stérile, comprenant une solution de NaCl à +4°C, et transporté à même température. Si les données cliniques du donneur sont conformes aux exigences relatives au don tissulaire, et que l'analyse sanguine de recherche de bactéries et virus se révèle négative, le tissu éligible peut subir le procédé de préparation.

Les vaisseaux sanguins du cordon ombilical sont identifiés et séparés les uns des autres par dissection.

Les vaisseaux sont découpés en segments. La longueur des segments est comprise entre 1 et 12 centimètres.

De manière préférée, la longueur d'un segment est comprise entre 1 et 6 centimètres lorsqu'il est constitué d'un segment d'artère ombilicale.

De manière préférée, la longueur d'un segment est comprise entre 3 et 12 centimètres lorsqu'il est constitué d'un segment de veine ombilicale.

Les segments de vaisseaux sont hydratés dans des bains d'eau purifiée, successifs, sous agitation douce, pendant environ 4 heures.

A l'issue de cette hydratation, la gelée de Wharton présente une souplesse qui facilite sa découpe.

Un mandrin, ou guide ou support rigide, de diamètre adapté est introduit dans la lumière du vaisseau destiné à former le guide pour la régénération tissulaire. Il est avantageusement utilisé comme mandrin, ou guide ousupport rigide, une aiguille à bout rond, avec un chas à une extrémité. Un diamètre adapté pour le mandrin, ou guide ou support rigide, est d'environ 1,5 millimètre pour une artère, et d'environ 3 millimètres pour une veine.

L'excès de gelée de Wharton entourant le vaisseau monté sur mandrin peut être enlevé au scalpel, de telle sorte qu'une épaisseur de 0,3 à 1 millimètre de gelée de Wharton demeure autour de la paroi pour une artère, et d'environ 1 à 1,5 millimètre pour une veine.

L'objet obtenu est défini comme un segment de veine ou artère ombilicale, hydraté à l'eau purifiée.

Le procédé de préparation d'un guide implantable non collabable est caractérisé en ce qu'il comprend au moins les étapes suivantes :
a) on dispose d'un segment de veine ou artère ombilicale, hydraté à l'eau purifiée ;
b) on procède au retournement de la paroi du segment en conservant de la gelée de Wharton pour obtenir un segment retourné ;
c) on insère un support rigide dans la lumière du segment retourné ;
d) on procède à une congélation à sec du segment retourné avec le support rigide
e) on procède à une décongélation du segment retourné et au retrait du support rigide ;
f) on effectue un traitement de viro-inactivation du segment retourné pour obtenir un segment viro-inactivé ;
g) on insère un support rigide dans la lumière du segment viro-inactivé ;
h) on effectue une lyophilisation du segment viro-inactivé avec le support rigide pour obtenir un segment viro-inactivé et lyophilisé ;
i) on retire le support rigide du segment viro-inactivé et lyophilisé pour obtenir le guide implantable non collabable.

Le procédé de préparation d'un guide implantable non collabable est, de préférence, caractérisé en ce que les étapes a), b), c), e), f), g) et i) s'effectuent à température ambiante.

Lors de l'étape b), le vaisseau est retourné après avoir suturé une extrémité de celui-ci au mandrin, ou support rigide. Il est avantageusement utilisé le chas d'une aiguille à bout rond pour suturer l'extrémité.

Selon un mode de réalisation préféré, une aiguille à bout pointu, et montée d'un fil de suture, est introduite à travers le chas de l'aiguille à bout rond, en traversant la partie inférieure du segment de veine. La veine peut ainsi être suturée au niveau du chas de l'aiguille.

Le procédé de préparation d'un guide implantable non collabable est, de préférence, caractérisé en ce que le segment retourné comprend dans sa lumière de la gelée de Wharton.

Selon un mode de réalisation préféré, le support rigide utilisé lors de l'étape c) est en PETG (polyéthylène téréphtalate glycolysé), ou en PET (polyéthylène téréphtalate), ou en PLGA (poly(lactic-co-glycolic acid)), ou en poly(α-hydroxy esters), notamment d'acide polylactique (PLA) et d'acide polyglycolique (PGA), ou différents polyéthylènes.

Selon un mode de réalisation préféré, le support rigide utilisé lors de l'étape c) est caractérisé par un diamètre de 1 à 3 millimètres pour une artère.

Selon un mode de réalisation préféré, le support rigide utilisé lors de l'étape c) est caractérisé par un diamètre de 2 millimètres pour une artère.

Selon un mode de réalisation préféré, le support rigide utilisé lors de l'étape c) est caractérisé par un diamètre de 3 ou 7 millimètres pour une veine, selon le diamètre de la veine.

Selon un mode de réalisation préféré, le support rigide utilisé lors de l'étape c) est caractérisé par un diamètre de 3 millimètres pour une veine.

Selon un mode de réalisation préféré, le support rigide utilisé lors de l'étape c) est caractérisé par un diamètre de 7 millimètres pour une veine.

Les étapes d) et e) assurent une lyse des cellules présentes dans le segment retourné par effet de pression osmotique.

Le support rigide utilisé lors de l'étape c) est ôté entre les étapes e) et f).

Afin de garantir l'innocuité du produit fini en tant que matériau implantable chez un patient, une première phase de traitement chimique f) est effectuée. En effet, le produit fini doit être exempt d'agents microbiologiques tels que bactéries, virus, parasites.

L'un des avantages techniques de retourner la paroi du segment est de permettre une optimisation du traitement de viro-inactivation et d'éliminer plus facilement les résidus de sang potentiel. En effet, le retournement de la paroi du segment a pour conséquence que la paroi qui était à l'origine au contact du sang circulant se retrouve désormais à l'extérieur du segment. Cette position permet un meilleur contact avec les solutions chimiques sous légère agitation de l'étape f).

Le procédé de préparation d'un guide implantable non collabable est, de préférence, caractérisé en ce que le traitement de viro-inactivation comprend au moins deux étapes successives de traitement par un agent de viro-inactivation chimique.

Par commodité, de manière non limitative, il sera fait référence aux étapes x et y pour désigner ces deux étapes successives de traitement par un agent de viro-inactivation chimique.

Le procédé de préparation d'un guide implantable non collabable est, de préférence, caractérisé en ce que l'agent de viro-inactivation chimique est choisi dans le groupe constitué par les alcools et/ou les peroxydes.

Le procédé de préparation d'un guide implantable non collabable est, de préférence, caractérisé en ce que la première étape de traitement x est effectuée par un agent de viro-inactivation choisi dans le groupe constitué par les alcools.

Selon un mode préféré de réalisation, le premier agent de viro-inactivation est l'éthanol à une teneur en alcool comprise entre 50 % et 80 % v/v.

Le procédé de préparation d'un guide implantable non collabable est, de préférence, caractérisé en ce que l'agent de viro-inactivation choisi dans le groupe constitué par les alcools est l'éthanol à 70 % v/v.

Le procédé de préparation d'un guide implantable non collabable est, de préférence, caractérisé en ce que la première étape de traitement x est effectuée pendant une durée de 60 minutes.

Le procédé de préparation d'un guide implantable non collabable est, de préférence, caractérisé en ce que le deuxième agent de viro-inactivation est le peroxyde d'hydrogène.

Le procédé de préparation d'un guide implantable non collabable est, de préférence, caractérisé en ce que le peroxyde d'hydrogène est sous une forme choisie parmi une solution aqueuse et un gaz.

Le procédé de préparation d'un guide implantable non collabable est, de préférence, caractérisé en ce que le peroxyde d'hydrogène est sous forme aqueuse dans une concentration comprise entre 3 % et 30 % w/v.

Le procédé de préparation d'un guide implantable non collabable est, de préférence, caractérisé en ce que la deuxième étape de viro-inactivation chimique y comporte deux sous-étapes de traitement, q et r, par le peroxyde d'hydrogène :
- une première sous-étape de traitement q s'effectuant avec une solution de peroxyde d'hydrogène à 30 % w/v pendant 15 minutes ;
- une deuxième sous-étape de traitement r s'effectuant avec une solution de peroxyde d'hydrogène à 3 % w/v pendant 60 minutes.

Le procédé de préparation d'un guide implantable non collabable est, de préférence, caractérisé en ce qu'il comporte en outre au moins une étape lors de laquelle le segment retourné est lavé à l'eau purifiée entre les étapes x et y de viro-inactivation chimique.

Le procédé de préparation d'un guide implantable non collabable est, de préférence, caractérisé en ce qu'il comporte en outre au moins une étape d'ajustement de pH de la solution dans laquelle se trouve le segment viro-inactivé entre les étapes f) et g).

Le procédé de préparation d'un guide implantable non collabable est, de préférence, caractérisé en ce qu'il comporte en outre au moins une étape d'ajustement de pH à 8,5 de la solution dans laquelle se trouve le segment viro-inactivé entre les étapes f) et g).

Le procédé de préparation d'un guide implantable non collabable est, de préférence, caractérisé en ce qu'il comporte en outre au moins une étape d'ajustage de pH de la solution dans laquelle se trouve le segment viro-inactivé, suivie d'au moins une étape de mise en tampon du segment viro-inactivé entre les étapes f) et g).

Selon un mode de réalisation préféré, cette au moins une étape de mise en tampon est définie par deux bains successifs de solution tampon de PBS (tampon phosphate salin).

Selon un mode de réalisation préféré, cette étape de mise en solution tampon est suivie de deux étapes de lavage en bains d'eau purifiée.

Suite à cette première étape f) de traitements chimiques, le segment peut être qualifié de viro-inactivé.

Selon un mode de réalisation préféré, le support rigide utilisé lors de l'étape g) est positionné dans la lumière du segment viro-inactivé.

Selon un mode de réalisation préféré, le support rigide utilisé lors de l'étape g) possède les mêmes caractéristiques que le support rigide utilisé lors de l'étape c).

Le segment viro-inactivé subit une étape h) qui est une lyophilisation. Cette étape permet à la fois d'assurer la qualité de conservation sous vide du produit obtenu selon le procédé, et d'assurer une étape supplémentaire de désinfection et de décellularisation au segment viro-inactivé, enfin d'obtenir un segment lyophilisé.

L'étape h) est caractérisée par la présence du support rigide introduit lors de l'étape g) dans la lumière avant la lyophilisation pour conserver l'ouverture, ce support rigide est enlevé après l'étape h) de lyophilisation.

Cette étape h) de lyophilisation, en combinaison avec l'étape f) de viro-inactivation, confère également au produit obtenu selon le procédé sa structure en réseau poreux longitudinal de sa surface interne.

Le procédé de préparation d'un guide implantable non collabable est, de préférence, caractérisé en ce que l'étape h) de lyophilisation est effectuée selon les étapes m et n suivantes :
m) une congélation en deux étapes m' et m" :
   - la première étape de congélation, m', s'effectuant à une température d'acclimatation choisie pour ne pas endommager l'intégralité structurelle, fonctionnelle et biologique du segment viro-inactivé ;
   - la deuxième étape de congélation, m", s'effectuant à la température finale de congélation qui est inférieure à la température d'acclimatation ;
n) une lyophilisation en deux étapes principales, dites primaire, n', et secondaire, n" :
   - l'étape de lyophilisation primaire n' s'effectuant par application d'un vide à environ 200 microbars et d'un profil de température ascendant ;
   - l'étape de lyophilisation secondaire n" s'effectuant par application d'un vide à environ 50 microbars et d'un profil de température descendant.

Le profil de température ascendant est avantageusement un profil selon lequel la température de lyophilisation est initialement réglée à une température initiale basse puis augmentée vers une température finale de lyophilisation primaire, en une ou plusieurs étapes de températures ascendantes intermédiaires. Le profil de température descendant est avantageusement un profil selon lequel la température de lyophilisation est initialement réglée à une température supérieure à la température finale de l'étape de lyophilisation primaire, puis qui est ensuite descendue vers une température finale de lyophilisation secondaire supérieure à la température initiale de l'étape de lyophilisation primaire.

Ces conditions de lyophilisation permettent d'obtenir un produit, qui est un segment viro-inactivé et lyophilisé, qui est notamment caractérisé en ce qu'il présente une teneur en eau résiduelle à température ambiante inférieure à 5%.

Selon un mode de réalisation préféré, le guide rigide utilisé lors de l'étape g) est ôté pour obtenir le guide implantable non collabable.

Selon un mode de réalisation préféré, le guide implantable non collabable est conditionné en conditions stériles.

Selon un mode de réalisation préféré, le guide implantable non collabable est conditionné en conditions stériles, en double sachets.

Selon un mode de réalisation préféré, le guide implantable non collabable, qui est emballé, subit une dernière étape de stérilisation qui est une irradiation gamma.

Le procédé de préparation d'un guide implantable non collabable est, de préférence, caractérisé en ce qu'il comporte en outre une étape de stérilisation du guide implantable non collabable viro-inactivé et lyophilisé, par exposition de ce dernier à des rayonnements gamma à 25-32 kGy après l'étape i).

Le procédé de préparation d'un guide implantable non collabable est caractérisé en ce que le taux des facteurs de croissance présents dans la gelée de Wharton (NGF, TGF, FGF) est préservé de préférence entre 70 et 80 %.

Le procédé de préparation d'un guide implantable non collabable est caractérisé en ce que le taux des facteurs de croissance présents dans la gelée de Wharton (NGF, TGF, FGF) est préservé de préférence entre 60 et 70 %.

Le procédé de préparation d'un guide implantable non collabable est caractérisé en ce que le taux des facteurs de croissance présents dans la gelée de Wharton (NGF, TGF, FGF) est préservé de préférence entre 50 et 60 %.

Le procédé de préparation d'un guide implantable non collabable est caractérisé en ce que le taux des facteurs de croissance présents dans la gelée de Wharton (NGF, TGF, FGF) est préservé de préférence entre 40 et 50 %.

Le procédé de préparation d'un guide implantable non collabable est caractérisé en ce que le taux des facteurs de croissance présents dans la gelée de Wharton (NGF, TGF, FGF) est préservé de préférence entre 30 et 40 %.

Le procédé de préparation d'un guide implantable non collabable est caractérisé en ce que le taux des facteurs de croissance présents dans la gelée de Wharton (NGF, TGF, FGF) est préservé de préférence entre 20 et 30 %.

Le procédé de préparation d'un guide implantable non collabable est caractérisé en ce que le taux des facteurs de croissance présents dans la gelée de Wharton (NGF, TGF, FGF) est préservé de préférence entre 10 et 20 %.

Le procédé de préparation d'un guide implantable non collabable est caractérisé en ce que le taux des facteurs de croissance présents dans la gelée de Wharton (NGF, TGF, FGF) est préservé de préférence entre 5 et 10 %.

Le produit obtenu selon le procédé peut être défini comme un guide implantable non collabable pour la régénération tissulaire, constitué d'un segment d'artère ou de veine ombilicale dont la paroi est retournée, caractérisé en ce qu'il est viro-inactivé et lyophilisé.

Le produit obtenu selon le procédé peut être défini comme un guide implantable non collabable pour la régénération tissulaire, également caractérisé en ce qu'il est implantable sans réhydratation préalable.

Le procédé précédemment décrit permet l'obtention d'un matériau d'allogreffe viro-inactivé et lyophilisé.

Le matériau d'allogreffe est présenté sous une forme utilisable comme greffe choisie dans le groupe constitué par la greffe de guide de régénération nerveuse, la greffe de guide de régénération tendineuse, la greffe de guide de régénération ligamentaire.

Le matériau d'allogreffe viro-inactivé et lyophilisé peut être utilisé en tant que greffe choisie dans le groupe constitué par la réparation d'un nerf du système nerveux périphérique, la réparation du tendon, la réparation du ligament.

Le matériau d'allogreffe viro-inactivé et lyophilisé peut être utilisé en chirurgie, notamment lors de l'utilisation d'une technique d'enveloppement ou une technique de manchonnage d'un tissu biologique lésé.

Lorsque le matériau d'allogreffe viro-inactivé et lyophilisé est utilisé en chirurgie lors de l'utilisation d'une technique d'enveloppement, le matériau d'allogreffe est coupé longitudinalement, par exemple à l'aide de ciseaux chirurgicaux, avant implantation.

Le matériau d'allogreffe viro-inactivé et lyophilisé peut être utilisé en chirurgie en le sectionnant éventuellement à sec, de telle sorte que la longueur du matériau soit d'au moins environ 1 centimètre supérieure à la longueur de l'intervalle à ponter entre les extrémités proximale et distale du tissu à réparer, qui peut être un nerf du système nerveux périphérique, un tendon ou un ligament. Le tissu à réparer est préparé de telle sorte que la section soit nette.

Le matériau d'allogreffe viro-inactivé et lyophilisé est manipulé à sec. Une extrémité (proximale ou distale) du tissu à réparer est insérée à l'intérieur du matériau d'allogreffe viro-inactivé et lyophilisé, et celui-ci est glissé sur l'extrémité du tissu.

Les deux extrémités du nerf, extrémité proximale et extrémité distale, sont suturées.

En cas de perte de substance sévère, le tissu à réparer est introduit dans l'allogreffe à chaque extrémité et stabilisé par suture, l'allogreffe jouant le rôle de guide.

Le matériau d'allogreffe viro-inactivé et lyophilisé est glissé au-delà de la suture, en le positionnant de part et d'autre de celle-ci, avec les rebords équidistants de la ligne de suture.

Le matériau d'allogreffe viro-inactivé et lyophilisé est naturellement réhydraté par les tissus organiques environnants et s'assouplit.

Comme précédemment indiqué, le guide implantable non collabable est notamment lyophilisé, il est donc manipulable à sec. Cette caractéristique confère notamment au matériau d'allogreffe une rigidité structurelle permettant une manipulation facilitée par le professionnel exerçant l'acte chirurgical d'implantation dudit matériau.

En effet, il est connu que le geste technique chirurgical présentement décrit nécessite une rigidité structurelle du matériau d'allogreffe, tel que défini dans la présente demande, de sorte à faciliter l'insertion d'une aiguille de suture courbe ou longue à l'intérieure de la structure, à permettre les mouvements de va-et-vient de ladite aiguille, et le glissement du matériau d'allogreffe le long du fil de suture puis le long du nerf en épousant sa forme sans déformation ou dénaturation significative de la structure tubulaire.

Ces avantages techniques conférés par un matériau d'allogreffe lyophilisé et manipulable à sec sont difficilement obtenus à partir d'un produit humide et souple dont la structure tubulaire sera collabable, c'est-à-dire qu'elle aura naturellement tendance à perdre sa forme tubulaire lors de la manipulation durant l'acte chirurgical d'implantation. En effet, lorsque par exemple une veine, qui est un produit humide et souple, est utilisée lors d'une technique de manchonnage, il est nécessaire de traverser la lumière de la veine avec une pince afin de saisir l'aiguille destinée à effectuer la suture. Ce geste est particulièrement difficile avec une veine qui aura naturellement tendance à se déformer lors de sa manipulation, ce qui implique un risque de détérioration du produit lors du passage de la pince et/ou de l'aiguille. La difficulté de ce geste ainsi que les risques associés s'accroissent avec l'accroissement de la longueur de la veine utilisée.

Ces avantages techniques conférés par un matériau d'allogreffe lyophilisé et manipulable à sec sont difficilement obtenus à partir d'un produit peu ou pas déformable tel que par exemple les neurotubes synthétiques ou en collagène, en ce que la structure tubulaire sera trop solide pour permettre le passage d'une aiguille de suture courbe à travers la lumière dudit neurotube.

Le matériau d'allogreffe viro-inactivé et lyophilisé peut également être utilisé lors de l'utilisation d'une technique d'enrobage, par exemple lorsque le nerf est lésé mais non sectionné ou lorsque l'accès au site d'implantation est difficile.

Lorsque le matériau d'allogreffe viro-inactivé et lyophilisé est utilisé lors d'une technique d'enrobage, il est préalablement coupé longitudinalement pour enrober le tissu lésé ou difficile d'accès, puis optionnellement suturé.

Ainsi, lors de l'utilisation du matériau d'allogreffe viro-inactivé et lyophilisé, le praticien peut décider à tout moment après la préparation du site receveur quelle est la meilleure technique à employer pour l'utilisation du produit selon l'invention, manchonnage ou enrobage, selon ses besoins techniques.

### Exemple 1 : Exemple d'un mode de réalisation de l'invention selon lequel un segment d'artère ombilicale est traité selon le procédé pour obtenir un guide implantable non collabable pour la régénération tissulaire.

Un donneur proprement informé et consentant en accord avec les exigences de la Déclaration d'Helsinki offre en don le cordon ombilical issu d'un accouchement. Par exigence sanitaire relative aux dons de tissus et de cellules d'origine humaine, une qualification en amont du donneur est systématique. Cette qualification implique une recherche de virus HIV, hépatites B, C, HTLV et de la bactérie tréponème pâle responsable de la syphilis.

Le cordon ombilical est récupéré le plus tôt possible en salle d'accouchement. Il est avantageusement placé dans une boite stérile, comprenant une solution de NaCl à +4°C.

Au laboratoire, en salle stérile, la procédure suivante est appliquée :
Les vaisseaux sanguins du cordon ombilical sont identifiés et séparés les uns des autres par dissection.

Une artère est découpée en un segment d'une longueur de 3 centimètres.

Le segment est rincé et hydraté dans des bains d'eau purifiée successifs, sous agitation douce, pendant 4 heures.

Une aiguille rigide à bout rond, d'un diamètre de 1,5 millimètre, disposant d'un chas à une extrémité, est insérée dans la lumière du segment de l'artère.

L'excès de gelée de Wharton entourant l'artère montée sur l'aiguille est enlevé au scalpel, de telle sorte qu'une épaisseur d'environ 0,3 millimètre de gelée de Wharton demeure autour de la paroi.

Une aiguille à bout pointu, et montée d'un fil de suture, est introduite à travers le chas de l'aiguille à bout rond, en traversant la partie inférieure du segment d'artère. L'artère est ainsi suturée au niveau du chas de l'aiguille.

Le segment fixé est retourné sur lui-même, de telle sorte que la paroi du segment où demeure la gelée de Wharton est désormais dans la lumière du segment obtenu. Le fil maintenant le segment à l'aiguille est ôté.

Un support rigide en PETG de 1,75 millimètre de diamètre, selon le diamètre de la veine, est positionné dans la lumière du segment retourné.

Le segment retourné et monté sur le support rigide est congelé à sec à une température de -80°C.

Lors du traitement chimique, le segment retourné et monté sur le guide rigide est décongelé à l'air libre, à température ambiante, pendant une durée de 5 minutes. Le support rigide en PETG est ôté. Cette étape de congélation, suivie d'une décongélation, assure une décellularisation importante du tissu biologique.

Le segment retourné subit une succession de bains assurant un traitement chimique de celui-ci. Ce traitement a pour objectif une désinfection de l'artère et de la gelée de Wharton, et tout particulièrement sa viro-inactivation. Une agitation linéaire douce du milieu liquide est appliquée lors de chaque bain afin d'assurer une pénétration homogène des solvants dans les tissus.

Dans un premier temps, le segment retourné est déposé dans un bain d'eau purifiée à température ambiante pendant environ 3 heures. Cette étape assure tant la fin de la décongélation du tissu physiologique, qu'une étape de lyse cellulaire par pression osmotique.

Puis, le segment retourné est transféré dans un bain décontaminant composé d'éthanol 70 % v/v à température ambiante pendant environ 1 heure.

Un lavage est effectué dans de l'eau purifiée pendant environ 15 minutes à température ambiante pour retirer l'éthanol.

Afin d'assurer la seconde étape de traitement décontaminant, le segment retourné est transféré dans un bain composé de peroxyde d'hydrogène à 30 % w/v à température ambiante pendant environ 15 minutes.

Puis, le segment retourné est transféré dans un bain décontaminant composé de peroxyde d'hydrogène à 3 % w/v à température ambiante pendant environ 1 heure. Le segment obtenu est viro-inactivé.

L'action chimique appliquée au segment viro-inactivé est ensuite neutralisée, dans au moins un bain comportant de l'hydroxyde de sodium dilué autour d'un pH de 8,5. Le traitement d'au moins un bain de neutralisation s'effectue à température ambiante pendant environ 15 minutes.

Afin d'assurer un rééquilibrage du pH et pour éliminer au mieux les résidus organiques se détachant du tissu d'intérêt, le segment viro-inactivé est transféré dans au moins un bain de tampon physiologique (PBS), afin d'assurer son rééquilibrage physiologique. Le au moins un bain s'effectue à température ambiante pendant environ 15 minutes.

Enfin, le segment viro-inactivé est transféré dans deux bains successifs à l'eau purifiée, à température ambiante, pendant au moins 15 minutes et jusqu'à environ 1 heure.

A cette étape du procédé, on peut considérer que le segment obtenu selon ce traitement chimique est un segment en grande partie désinfecté, notamment viro-inactivé, et décellularisé.

Suite à cette partie relative aux traitements chimiques, le segment viro-inactivé subit une lyophilisation.

Un support rigide en PETG stérile est inséré dans la lumière du segment viro-inactivé. Ce dernier est placé sur un plateau en inox.

L'ensemble est transféré dans un lyophilisateur, où une étape de congélation suivie d'une étape de lyophilisation sont effectuées selon les modalités suivantes :
- Congélation :
   - La première étape de congélation s'effectue à une température d'acclimatation choisie pour ne pas endommager l'intégrité structurelle, fonctionnelle et biologique du segment viro-inactivé ;
   - la deuxième étape de congélation s'effectue à la température finale de congélation qui est inférieure à la température d'acclimatation ;
- Lyophilisation :
   - Une étape de lyophilisation primaire s'effectue par application d'un vide à environ 200 microbars et par application d'un profil de température ascendant ;
   - Une étape secondaire de lyophilisation s'effectue par application d'un vide à environ 50 microbars et par application d'un profil de température descendant.

Suite à cette étape de lyophilisation, le segment retourné est un segment désinfecté, notamment viro-inactivé, décellularisé et lyophilisé.

Le support rigide en PETG est ôté pour obtenir le guide implantable non collabable.

Le guide implantable non collabable est conditionné en conditions stériles, par exemple en double sachets.

Une dernière étape de stérilisation du guide implantable non collabable est effectuée par exposition de celui-ci à des rayonnements gamma à 25-32 kGrays.

Le produit ainsi obtenu est utilisé comme matériau d'allogreffe, par exemples la greffe de guide de régénération nerveuse, la greffe de guide de régénération tendineuse, la greffe de guide de régénération ligamentaire.

Quelques utilisations envisageables du produit obtenu selon le procédé sont donc la réparation d'un nerf du système nerveux périphérique, la réparation du tendon, la réparation du ligament.

### Exemple 2 : Exemple d'un mode d'utilisation d'un guide implantable non collabable pour la régénération tissulaire selon l'invention.

On utilise comme modèle expérimental un rat de la lignée Sprague Dawley, âgé de 13 semaines.

On effectue une anesthésie générale de l'animal, et la procédure analgésique est délivrée 30 minutes à 1 heure avant chirurgie.

Après dissection du septum intermusculaire entre le muscle fessier maximal et le biceps fémoral, afin d'exposer et dissocier le nerf sciatique, on procède à une transsection de ce nerf chez l'animal.

Cette transsection est réalisée de telle sorte qu'un segment de 1 cm du nerf sciatique exposé est enlevé sous grossissement de 4x en utilisant des micros-ciseaux.

Un guide implantable non collabable pour la régénération tissulaire selon l'invention est coupé pour atteindre une longueur de 2 centimètres.

Le guide implantable non collabable est manipulé à sec, on insère le moignon de l'extrémité proximal du nerf dans la lumière du guide.

On suture le nerf par deux points épi-périneuraux, puis le guide est glissé au-delà de la suture nerveuse, en le positionnant de part et d'autre de celle-ci, avec un débord équidistant de la ligne de suture.

Après la procédure chirurgicale, le nerf sciatique est replacé entre les deux muscles, et la plaie cutanée est suturée à l'aide d'agrafes chirurgicales.

### Exemple 3 : Démonstration de l'efficacité de la viro-inactivation du guide implantable non collabable pour la régénération tissulaire selon l'invention.

Des mesures de la charge virale ont été effectuées dans des segments d'artères ombilicales dont les parois ont été retournées en conservant de la gelée de Wharton dans la lumière du segment, par injection de virus dans la gelée de Wharton afin de vérifier la viro-inactivation des tissus selon le procédé.

Les artères ombilicales étant caractérisées par une paroi plus épaisse que celle des veines ombilicales, la démonstration est plus probante avec du tissu biologique d'origine artérielle.

Les virus ont été inoculés dans la gelée de Wharton par une incision à la pince des parois vasculaires des segments de vaisseaux ombilicaux retournés à trois endroits.

Des aliquotes des segments de vaisseaux ombilicaux retournés ainsi traités sont prélevées afin d'être mises en solution et centrifugées. La fraction soluble sert à ensemencer des cellules en milieu de culture cellulaire. Chaque type de virus étudié est ensemencé sur le type adéquat de cellules afin de permettre l'expression de sa virulence.

Les virus ayant fait l'objet d'une mesure sont le virus Parvovirus porcin (ci-après noté PPV) et le virus Pseudorabies (ci-après noté PRV) impliqué dans la maladie d'Aujeszky. Le PPV est un virus nu, de petite dimension, considéré comme fortement résistant. Le PRV est un virus enveloppé considéré comme ayant une résistance physico-chimique moyenne.

Les mesures de charge virale ont été effectuées par détection de production virale dans des cellules infectées, et plus particulièrement par observation d'un effet spécifique cytopathique. Les virus injectés dans la gelée de Wharton, dans la lumière des segments de vaisseaux ombilicaux retournés, ont subi soit l'étape de traitement chimique à l'éthanol 70 % v/v selon le procédé, soit l'étape de traitement chimique au peroxyde d'hydrogène à 30 % w/v puis 3 % w/v selon le procédé.

La résistance du virus PRV a été évaluée par l'étape de traitement chimique à l'éthanol, ainsi que par l'étape de traitement chimique au peroxyde d'hydrogène. La résistance du virus PPV a été évaluée par l'étape de traitement chimique au peroxyde d'hydrogène.

Chaque mesure de la charge virale a été effectuée deux fois pour chaque essai, celles-ci sont notées dans le tableau suivant par les indications Run A et Run B.

Les résultats dans le tableau suivant représentent la diminution de la charge virale mesurée, exprimée en log. Cette diminution est mesurée par comparaison de la charge virale des aliquotes des segments de vaisseaux ombilicaux retournés avant, puis après le traitement indiqué.

| **Virus** | | **PRV** | |
|---|---|---|---|
| Ethanol 70 % pendant 1 heure | Run | **Facteurs de réduction moyenne (en 10g₁₀) sur le produit** | |
| | Echantillon | Vaisseaux retournés et gelée de Wharton | |
| | Essai | Infectivité | |
| | Run A | **>4,35** | |
| | Run B | **>4,53** | |
| **Virus** | | **PRV** | **PPV** |
| H₂O₂ (30 % pendant 15 minutes ; puis 3 % pendant 1 heure) | Run | **Facteurs de réduction moyenne (en log₁₀) sur le produit** | |
| | Echantillon | Vaisseaux retournés et gelée de Wharton | |
| | Essai | Infectivité | Infectivité |
| | Run A | **> 6,29** | **= 6,56** |
| | Run B | **= 5,71** | **= 5,93** |

L'efficacité des traitements chimiques selon le procédé, appliqués aux segments de vaisseaux ombilicaux retournés, et destinés à la viro-inactivation peut être constatée.

Le traitement chimique au peroxyde d'hydrogène est efficace contre le PPV et le PRV.

Le traitement chimique à l'éthanol est efficace contre le PPV et le PRV.

Comme démontré, la combinaison des deux traitements chimiques permet d'assurer une viro-inactivation des segments de vaisseaux ombilicaux retournés, tant des virus enveloppés que des virus nus. Cette viro-inactivation étant définie par une diminution de la population virale d'au moins 4 log.

### Exemple 4 : Démonstration de la préservation des caractéristiques structurelles du guide implantable non collabable pour la régénération tissulaire selon l'invention après réhydratation.

Les essais sont réalisés à partir de deux guides implantables pour la régénération tissulaire selon l'invention, dont l'un est obtenu à partir d'un segment de veine ombilicale (identifié comme « Veine ») et l'autre à partir d'un segment d'artère de cordon ombilical (identifié comme « Artère »).

Les étapes d'obtention des deux guides implantables pour la régénération tissulaire selon l'invention sont similaires à celles décrites à l'exemple 1 mais à partir de ces deux types de segments.

L'objectif des essais de réhydratation est d'observer l'évolution structurelle des guides implantables selon l'invention dans le temps lors de leurs réhydratations.

Chaque échantillon est pesé et mesuré (diamètre interne et diamètre externe, longueur) avant réhydratation.

Chaque échantillon est immergé dans un récipient comprenant environ 50 mL d'eau. Après 5 minutes, 10 minutes, 15 minutes, 30 minutes, 1 heure et 24 heures de réhydratation, les guides sont pesés et mesurés. Si le guide apparait comme trop imbibé d'eau avant mesure et pesée, l'eau en excès est ôtée avec un papier absorbant par capilarité.

Les résultats sont présentés dans le tableau suivant.

| Temps | Artère | | | | Veine | | | |
|---|---|---|---|---|---|---|---|---|
| | P (mg) | L(cm) | DI (mm) | DE (mm) | P (mg) | L (cm) | DI (mm) | DE (mm) |
| Initial | 7,6 | 2,060 | 1,75 | 3,55 | 82,2 | 2,880 | 5,1 | 8,55 |
| 5 minutes | 84,4 | 2,165 | 1,85 | 2,90 | 907,0 | 3,000 | 5,3 | 7,35 |
| 10 minutes | 81,3 | 2,100 | 1,80 | 2,70 | 878,3 | 3,350 | 4,45 | 7,30 |
| 15 minutes | 83,9 | 2,075 | 2,20 | 3,0 | 819,5 | 2,910 | 5,50 | 7,05 |
| 30 minutes | 101,3 | 2,150 | / | / | 819,1 | 2,805 | / | / |
| 1 heure | 96,2 | 2,150 | / | / | 795,9 | 2,865 | / | / |
| 24 heures | 85,0 | 2,175 | 1,90 | 2,75 | 812,9 | 2,945 | 5,50 | 7,80 |

| | | | | | | | | |
|---|---|---|---|---|---|---|---|---|
| P: Poids ; L: Longueur ; DI: Diamètre interne ; DE : Diamètre externe | | | | | | | | |

De part les faibles dimensions du produit, mesurer les diamètres internes et externes est difficile. Du produit initial lyophilisé jusqu'au produit réhydraté à 15 minutes, les valeurs sont stables avec une faible augmentation du diamètre interne et une faible diminution du diamètre externe. Ces variations de mesures sont probablement dues à l'incertitude liée à la mesure de faibles dimensions sur des tissus flexibles, plutôt que par une quelconque évolution des dimensions. De fait, ces paramètres de diamètres internes et diamètres externes n'ont pas été mesurés à 30 minutes et 1 heure. Les mesures finales après 24 heures de réhydratation confirment la stabilité de ces paramètres.

Concernant le paramètre des longueurs, la même observation est effectuée, les longueurs ne varient dans le temps de réhydratation que d'un maximum de 1 mm. Cette variation des longueurs n'est probablement pas un accroissement mais est probablement générée de nouveau par l'incertitude des mesures effectuées sur un tissu flexible.

Le poids est un paramètre plus fluctuant en ce que le produit initial est lyophilisé. Après 5 minutes de réhydratation, les guides implantables selon l'invention (veine et artère) ont atteint 80 % de leur poids maximal. Après 24 heures de réhydratation, les résultats demeurent stables (84 % du poids maximal pour l'artère et 90 % pour la veine).

Une étude qualitative a été menée en parallèle afin d'observer le comportement structurel du produit pendant la réhydratation.

Après 5 minutes de réhydratation, le produit obtenu à partir de l'artère apparaît translucide. Le guide implantable non collabable selon l'invention est flexible mais le lumen demeure ouvert. Les mêmes observations sont effectuées à chaque étape de mesure jusqu'à la mesure à 1 heure de réhydratation incluse. Après 24 heures de réhydratation, le lumen est sensiblement moins ouvert et un début d'aplatissement du guide implantable non collabable est observé et la forme tubulaire est altérée.

Après 5 minutes de réhydratation, le produit obtenu à partir de la veine apparaît blanchâtre. Le guide implantable non collabable selon l'invention est flexible mais le lumen demeure ouvert. Les mêmes observations sont effectuées à chaque étape de mesure jusqu'à la mesure à 24 heure de réhydratation incluse. A 24 heure, à la fin de l'essai, le lumen est toujours ouvert et la forme tubulaire du guide implantable non collabable est préservée.

Les guides implantables selon l'invention, obtenus à partir d'une veine ou d'une artère du cordon ombilical, sont réhydratés après seulement 5 minutes d'immersion dans l'eau (plus de 80 % du poids maximal atteint). Les dimensions (longueur, diamètre externe et diamètre interne) des guides ne sont pas impactées par la réhydratation.

Après 1 heure de réhydratation, les guides implantables selon l'invention, obtenus à partir d'une veine ou d'une artère du cordon ombilical, préservent leurs structures tubulaires avec un lumen ouvert. Les mêmes caractéristiques sont observées après 24 heures pour le guide implantable non collabable selon l'invention obtenu à partir d'une veine de cordon ombilical. Pour le guide implantable non collabable selon l'invention obtenu à partir d'une artère de cordon ombilical, à 24 heures un début d'aplatissement est observé ainsi qu'une légère obstruction du lumen.

Le guide implantable non collabable pour la régénération tissulaire, constitué d'un segment d'artère ou de veine ombilicale dont la paroi est retournée, caractérisé en ce qu'il est viro-inactivé et lyophilisé est donc implantable avec ou sans réhydratation préalable, une stabilité de la structure est observée pendant au moins 24 heures durant la réhydratation du produit.

### Exemple 5 : Démonstration de la préservation de la forme tubulaire du guide implantable non collabable pour la régénération tissulaire selon l'invention après réhydratation

Les essais sont réalisés à partir de trois guides implantables pour la régénération tissulaire selon l'invention, dont l'un est obtenu à partir d'un segment de veine ombilical (identifié comme « Veine ») et les deux autres à partir d'un segment d'artère de cordon ombilical (identifiés comme « Artère 1 » et « Artère 2 »). L'échantillon Veine a une longueur de 3 cm, l'échantillon Artère 1 une longueur de 1 cm et l'échantillon Artère 2 a une longueur de 2,8 cm.

Les étapes d'obtention des trois guides implantables pour la régénération tissulaire selon l'invention sont similaires à celles décrites à l'exemple 1 mais à partir de ces deux types de segments.

L'objectif des essais de réhydratation est de déterminer si les guides implantables selon l'invention préservent leurs formes tubulaires ou s'ils se ré-inversent lorsqu'ils sont longitudinalement coupés et réhydratés sur un mandrin.

En effet, la paroi du tissu biologique destiné à être traité selon l'invention, qui est un segment de veine ou d'artère, est retournée lors du procédé de production des guides implantables selon l'invention, de telle sorte que la paroi de la lumière du segment ainsi obtenu soit la paroi externe du vaisseau avant retournement.

Ainsi, il pourrait être préjugé qu'une section longitudinale des guides implantables selon l'invention induise un retour à la forme d'origine du produit avant son obtention selon le procédé, et donc un inversement de la forme tubulaire de la paroi.

Le protocole suivant est utilisé pour chaque échantillon : le guide implantable non collabable selon l'invention est coupé longitudinalement avec des ciseaux puis placé autour d'un mandrin en PETG de telle sorte à simuler l'emballage (wrapping en langue anglaise) autour d'un tissu, par exemple un nerf lésé. L'ensemble est déposé dans un récipient. A l'aide d'une pipette, le guide implantable non collabable selon l'invention est réhydraté jusqu'à totale immersion. L'apparence du guide implantable non collabable réhydraté et la quantité d'eau sont notées. La réhydratation est réalisée par tranches de 50 µL d'ajouts en eau et 5 minutes d'intervalle sont respectés entre chaque apport en eau. Lorsque le guide implantable non collabable est totalement réhydraté, il est immergé dans l'eau et la durée de l'immersion est notée.

Les résultats suivants sont obtenus pour les échantillons Artère 1 et Artère 2 :

| | Echantillon Artère 1 | Echantillon Artère 2 |
|---|---|---|
| Volume d'eau (µL) | Apparence | Apparence |
| 50 | Commence à devenir translucide Réhydratation incomplète Aucune inversion | Réhydratation incomplète Aucune inversion |
| 100 | Translucide et ramolli Aucune inversion | Commence à devenir translucide Réhydratation incomplète Aucune inversion |
| 150 | Réhydratation complète Aucune inversion | Translucide et ramolli Aucune inversion |
| 250 | / | Réhydratation complète Aucune inversion |

Après immersion totale, aucune inversion n'est observée, les guides implantables selon l'invention conservent leur forme tubulaire même après section longitudinale et réhydratation complète. Après 18 heures en immersion total, les guides implantables selon l'invention conservent leur forme tubulaire et demeurent sur le mandrin. La réhydratation n'impacte pas la forme.

En conséquence, les guides implantables selon l'invention obtenus à partir de tissus biologiques qui sont des artères ombilicales sont adaptés pour être utilisés en tant qu'enveloppes (wraps en langue anglaise) et suturés en milieu humide.

Les résultats suivants sont obtenus pour l'échantillon Veine :

| Echantillon Veine | |
|---|---|
| Volume en eau (µL) | Apparence |
| 50 (µL) | Réhydratation incomplète Aucune inversion |
| 100 | |
| 150 | |
| 200 | |
| 250 | Commence à devenir ramolli Réhydratation incomplète Aucune inversion |
| 300 | |
| 350 | |
| 400 | |
| 450 | Commence à devenir translucide Ramolli Aucune inversion |
| 500 | |
| 550 | |
| 600 | Réhydratation complète Aucune inversion |

Après 1 heure d'immersion totale, l'échantillon Veine n'est pas inversé et conserve sa forme tubulaire.

En conséquence, les guides implantables selon l'invention obtenus à partir de tissus biologiques qui sont des veines ombilicales sont également adaptés pour être utilisés en tant qu'enveloppes (wraps en langue anglaise) et suturés en milieu humide.

En conclusion, les guides implantables selon l'invention peuvent être utilisés dans des techniques d'enveloppement (wrapping en langue anglaise) pour entourer des tissus à réparer, par exemple des nerfs lésés. Une fois coupés longitudinalement et positionnés autour du tissu dont la régénération est recherchée, les guides implantables selon l'invention préservent leur forme tubulaire en environnement humide durant au moins 18 heures, conférant le temps nécessaire pour effectuer la suture.

Contrairement à ce qui pourrait être préjugé, le coupure longitudinale n'induit pas l'inversion du produit de telle sorte que celui-ci recouvrirait sa forme d'origine avant inversion lors du procédé.

## Revendications

1. Guide implantable non collabable pour la régénération tissulaire, constitué d'un segment d'artère ou de veine ombilicale dont la paroi est retournée, **caractérisé en ce qu'**il est viro-inactivé et lyophilisé.

2. Guide implantable non collabable selon la revendication 1, **caractérisé en ce qu'**il comprend dans sa lumière de la gelée de Wharton.

3. Guide implantable non collabable selon l'une quelconque des revendications précédentes, **caractérisé en ce que** le diamètre de la lumière du segment est compris entre 1 et 7 millimètres.

4. Guide implantable non collabable selon l'une quelconque des revendications précédentes, **caractérisé en ce que** la longueur du segment est comprise entre 1 et 12 centimètres.

5. Guide implantable non collabable selon l'une quelconque des revendications précédentes, **caractérisé en ce qu'**il est destiné à être utilisé comme matériau d'allogreffe choisie dans le groupe constitué par la greffe de guide de régénération nerveuse, la greffe de guide de régénération tendineuse, la greffe de guide de régénération ligamentaire.

6. Procédé de préparation d'un guide implantable non collabable selon l'une quiconque des revendications précédentes, **caractérisé en ce qu'**il comprend au moins les étapes suivantes :
a) on dispose d'un segment de veine ou artère ombilicale, hydraté à l'eau purifiée ;
b)on procède au retournement de la paroi du segment en conservant de la gelée de Wharton pour obtenir un segment retourné ;
c) on insère un support rigide dans la lumière du segment retourné ;
d)on procède à une congélation à sec du segment retourné avec le support rigide ;
e)on procède à une décongélation du segment retourné et au retrait du support rigide ;
f) on effectue un traitement de viro-inactivation du segment retourné pour obtenir un segment viro-inactivé ;
g) on insère un support rigide dans la lumière du segment viro-inactivé ;
h) on effectue une lyophilisation du segment viro-inactivé avec le support rigide pour obtenir un segment viro-inactivé et lyophilisé ;
i) on retire le support rigide du segment viro-inactivé et lyophilisé pour obtenir le guide implantable non collabable.

7. Procédé de préparation d'un guide implantable non collabable selon la revendication 6, **caractérisé en ce que** le segment retourné comprend dans sa lumière de la gelée de Wharton.

8. Procédé de préparation d'un guide implantable non collabable selon l'une quelconque des revendications 6 et 7, **caractérisé en ce que** le traitement de viro-inactivation comprend au moins deux étapes successives de traitement par un agent de viro-inactivation chimique.

9. Guide implantable non collabable selon l'une quelconque des revendications 1 à 5 ou susceptible d'être obtenu selon le procédé de l'une quelconque des revendications 6 à 8 précédentes, présenté sous une forme utilisable comme greffe choisie dans le groupe constitué par la greffe de guide de régénération nerveuse, la greffe de guide de régénération tendineuse, la greffe de guide de régénération ligamentaire.

## Patentansprüche

1. Nichtkollabierbare implantierbare Führung für die Geweberegeneration, bestehend aus einem Nabelarterien- oder Nabelvenenabschnitt, deren Wand umgedreht ist, **dadurch gekennzeichnet, dass** sie virus-inaktiv und lyophilisiert ist.

2. Nichtkollabierbare implantierbare Führung nach Anspruch 1, **dadurch gekennzeichnet, dass** sie in ihrem Lumen Wharton-Gelee umfasst.

3. Nichtkollabierbare implantierbare Führung nach einem der vorangehenden Ansprüche, **dadurch gekennzeichnet, dass** der Durchmesser des Lumens des Abschnitts zwischen 1 und 7 Millimeter beträgt.

4. Nichtkollabierbare implantierbare Führung nach einem der vorangehenden Ansprüche, **dadurch gekennzeichnet, dass** die Länge des Abschnitts zwischen einem und zwölf Zentimeter beträgt.

5. Nichtkollabierbare implantierbare Führung nach einem der vorangehenden Ansprüche, **dadurch gekennzeichnet, dass** sie dazu bestimmt ist, als Allotransplantatmaterial verwendet zu werden, das ausgewählt ist aus der Gruppe bestehend aus Führungstransplantat für Nervenregeneration, Führungstransplantat für Sehnenregeneration, Führungstransplantat für Bänderregeneration.

6. Verfahren zum Herstellen einer nichtkollabierbaren implantierbaren Führung nach einem der vorangehenden Ansprüche, **dadurch gekennzeichnet, dass** es mindestens die folgenden Schritte umfasst:
a) Bereitstellen eines in gereinigtem Wasser hydratisierten Abschnitts einer Nabelvene oder einer Nabelarterie;
b) Fortfahren mit dem Umdrehen der Wand des Abschnitts unter Beibehalten des Wharton-Gelees, um einen umgedrehten Abschnitt zu erhalten;
c) Einführen eines starren Trägers in das Lumen des umgedrehten Abschnitts;
d) Fortfahren mit Gefriertrocknen des umgedrehten Abschitts mit dem starren Träger;
e) Fortfahren mit Auftauen des umgedrehten Abschnitts und Entfernen des starren Trägers;
f) Vornehmen einer Virusinaktivierungsbehandlung des umgedrehten Abschitts, um einen virus-inaktivierten Abschnitt zu erhalten;
g) Einführen eines starren Trägers in das Lumen des virus-inaktivierten Abschnitts;
h) Lyophilisiern des virus-inaktivierten Abschnittes mit dem starren Träger, um einen virus-inaktivierten und lyophilisierten Abschnitt zu erhalten;
i) Zurückziehen des starren Trägers aus dem virus-inaktivierten und lyophilisierten Abschnitt, um die nichtkollabierbare implantierbare Führung zu erhalten.

7. Verfahren zum Herstellen einer nichtkollabierbaren implantierbaren Führung nach Anspruch 6, **dadurch gekennzeichnet, dass** der umgedrehte Abschnitt in seinem Lumen Wharton-Gelee umfasst.

8. Verfahren zum Herstellen einer nichtkollabierbaren implantierbaren Führung nach einem der Ansprüche 6 und 7, **dadurch gekennzeichnet, dass** die Virusinaktivierungsbehandlung wenigstens zwei aufeinanderfolgende Behandlungschritte mit einem chemischen Virusinaktivierungsmittel umfasst.

9. Nichtkollabierbare implantierbare Führung nach einem der Ansprüche 1 bis 5 oder erhältlich gemäß dem Verfahren nach einem der Ansprüche 6 bis 8, vorliegend in einer als Transplanatat geeigneten Form, das ausgewählt ist aus der Gruppe bestehend aus Führungstransplantat für Nervenregeneration, Führungstransplantat für Sehnenregeneration, Führungstransplantat für Bänderregeneration.

## Claims

1. Non-collapsible implantable tissue regeneration guide, consisting of a segment of an umbilical artery or vein whose wall is inverted, **characterized in that** it is viro-inactivated and freeze-dried.

2. Implantable non-collapsible guide according to Claim 1, **characterized in that** it comprises Wharton's jelly within its lumen.

3. Non-collapsible implantable guide according to any of the preceding claims, **characterized in that** the diameter of the lumen of the segment is from 1 to 7 millimeters.

4. Non-collapsible implantable guide according to any of the preceding claims, **characterized in that** the length of the segment is from 1 to 12 centimeters.

5. Non-collapsible implantable guide according to any of the preceding claims, **characterized in that** it is intended to be used as an allograft material selected from the group consisting of the nerve regeneration guide graft, the tendon regeneration guide graft, the ligament regeneration guide graft.

6. Method for preparing a non-collapsible implantable guide, according to any of the preceding claims, **characterized in that** it comprises at least the following steps:
a) obtaining a segment of vein or umbilical artery, hydrated with purified water;
b) inverting the wall of the segment while retaining the Wharton's jelly to obtain an inverted segment;
c) inserting a rigid support into the lumen of the inverted segment;
d) dry freezing the inverted segment with the rigid support;
e) thawing the inverted segment and remove the rigid support;
f) performing a viro-inactivation treatment of the inverted segment to obtain a viro-inactivated segment;
g) inserting a rigid support into the lumen of the viro-inactivated segment;
h)dry freezing the viro-inactivated segment with the rigid support to obtain a viro-inactivated and freeze dried segment;
i) removing the rigid support from the viro-inactivated and freeze-dried segment to obtain the non-collapsible implantable guide.

7. Method for preparing an non-collapsible implantable guide according to claim 6, **characterized in that** the inverted segment comprises Wharton's jelly within its lumen.

8. Method for preparing a non-collapsible implantable guide according to any one of claims 6 and 7, **characterized in that** the viro-inactivation treatment comprises at least two successive steps of treatment with a chemical viro-inactivation agent.

9. Non-collapsible implantable guide according to any one of claims 1 to 5 or suceptible to be obtained according to the method of any one of the preceding claims 6 to 8, presented in a useable form as a graft selected from the group consisting of the nerve regeneration guide graft, tendon regeneration guide graft, ligament regeneration guide graft.
